# EUROPÄISCHE PATENTSCHRIFT

(11) **EP 0 749 438 B1**
(45) Veröffentlichungstag und Bekanntmachung des Hinweises auf die Patenterteilung: **13.12.2000**
(21) Anmeldenummer: 95912217.7
(22) Anmeldetag: 07.03.1995
(51) Int. Cl.: C07J 71/00, A61K 31/58

(54) **NEUE SILYLVERBINDUNGEN UND IHRE VERWENDUNG**
NOVEL SILYL COMPOUNDS AND THEIR USE
NOUVEAUX COMPOSES SILYLE ET LEUR UTILISATION

(30) Priorität: 09.03.1994 CH 70194
(43) Veröffentlichungstag der Anmeldung: 27.12.1996
(73) Patentinhaber: Byk Gulden Lomberg Chemische Fabrik GmbH, 78467 Konstanz (DE)
(72) Erfinder: AMSCHLER, Hermann, D-78315 Radolfzell (DE); FLOCKERZI, Dieter, D-78476 Allensbach (DE); GUTTERER, Beate, D-78476 Allensbach (DE)
(86) Internationale Anmeldenummer: EP9500836
(87) Internationale Veröffentlichungsnummer: WO9524416

(56) Entgegenhaltungen:
- WO-A-92/11280
- US-A- 3 996 359
- CHEMICAL ABSTRACTS, vol. 106, no. 9, 2.März 1987 Columbus, Ohio, US; abstract no. 67573q, L. CASTELLET ET AL 'Process for the preparation of budesonide.' Seite 641; Spalte 1; & ES-A-543 211 (FARMHISPANIA S. A.)

## Beschreibung

### Anwendungsgebiet der Erfindung

Die Erfindung betrifft neue Silylverbindungen und ihre Verwendung bei der Synthese von Wirkstoffen, die in der pharmazeutischen Industrie zur Herstellung von Arzneimitteln verwendet werden.

### Bekannter technischer Hintergrund

Aus dem U.S. Patent 3,513,163 sind 21-Trialkylsiloxy-pregnanderivate bekannt, die entzündungshemmende und gluconeogenetische Eigenschaften besitzen sollen. In der DE-OS 41 29 535 werden Pregna-1,4-dien-3,20-dion-16,17-acetal-21-ester offenbart, die am cyclischen Acetalring einen Butyl-, Isopropyl-, sec.-Butyl-, Cyclohexyl- oder Phenylrest tragen, und deren C-21-Hydroxylgruppe durch einen Acetyl- oder Isobutyrylrest acyliert ist.

WO-A-92/11280 offenbart ein Verfahren zur Herstellung von Verbindungen gemäß der vorliegenden Formel (I) durch Kondensation von 16-alpha-Hydroxy-21-acetyl-prednisolon mit Butyraldehyd, Kristallisation des Produkts aus Ethanol und Verseifung des Acetats.

### Beschreibung der Erfindung

Bei chiralen Wirkstoffen ist das eine Enantiomer bzw. das eine Epimer oftmals wirksamer oder mit weniger Nebenwirkungen verknüpft als das andere. Die möglichst selektive und reine Gewinnung des gewünschten Enantiomers bzw. Epimers ist daher bei chiralen Wirkstoffen von großer Bedeutung.

Erfindungsgemäß wird nun ein neues Verfahren zur Verfügung gestellt, mit dem sich die Epimeren bestimmter Pregna-1,4-dien-3,20-dionderivate besonders gut trennen lassen.

Gegenstand der Erfindung ist ein Verfahren zur Anreicherung des R-Epimeren in einem R/S-Epimerengemisch von Verbindungen der Formel I (siehe beigefügtes Formelblatt), worin R1 1-7C-Alkyl oder 3-8C-Cycloalkyl bedeutet, das dadurch gekennzeichnet ist, daß man das R/S-Epimerengemisch der Verbindungen der Formel I mit Verbindungen X-Si(R2)(R3)R4, worin R2, R3 und R4 gleich oder verschieden sind und jeweils einen 1-7C-Alkylrest oder Phenylrest darstellen und X eine geeignete Abgangsgruppe darstellt, silyliert, das erhaltene R/S-Gemisch des Silylderivats der Formel II (siehe beigefügtes Formelblatt), worin R1, R2, R3 und R4 die oben angegebenen Bedeutungen haben, fraktioniert zur Kristallisation bringt, und aus den zuerst erhaltenen Kristallfraktionen das mit R-Epimeren angereicherte R/S-Epimerengemisch von Verbindungen der Formel I durch saure Hydrolyse freisetzt.

1-7C-Alkyl steht für geradkettige oder verzweigte Alkylreste mit 1 bis 7 Kohlenstoffatomen. Beispielsweise seien der Heptyl-, Hexyl-, Neopentyl-, Isopentyl-, Pentyl-, Butyl-, iso-Butyl-, sec.-Butyl-, tert.-Butyl-, Propyl-, Isopropyl-, Ethyl- und der Methylrest genannt.
Ein bevorzugter 1-7C-Alkylrest R1 ist der Propylrest.
Ein bevorzugter 1-7C-Alkylrest R2 ist der Methylrest.
Ein bevorzugter 1-7C-Alkylrest R3 ist der Methylrest.
Ein bevorzugter 1-7C-Alkylrest R4 ist der 1,1,2-Trimethylpropylrest (Thexylrest).

3-8C-Cycloalkyl steht für den Cyclopropyl-, Cyclobutyl-, Cyclopentyl-, Cyclohexyl-, Cycloheptyl- und Cyclooctylrest. Ein bevorzugter 3-8C-Cycloalkylrest ist der Cyclohexylrest.

Die Umsetzung der Verbindungen der Formel I mit den Silylverbindungen X-Si(R2)(R3)R4 erfolgt auf eine dem Fachmann an sich vertraute Weise in inerten Lösungsmitteln, wie beispielsweise in Dimethylformamid, Chloroform, Methylenchlorid, Diethylether, Tetrahydrofuran oder Pyridin bei Temperaturen zwischen 20°C und 80°C, insbesondere zwischen 40°C und 60°C. Als geeignete Abgangsgruppen X seien vorzugsweise Halogenatome, insbesondere Chlor genannt.

Die Umsetzung erfolgt vorzugsweise in Gegenwart einer Hilfsbase, beispielsweise in Gegenwart eines anorganischen Carbonats, wie Kaliumcarbonat, oder in Gegenwart eines geeigneten organischen Amins, wie Triethylamin, Diisopropylethylamin, Pyridin oder Imidazol.

Die fraktionierte Kristallisation wird auf eine dem Fachmann vertraute Weise vorgenommen, beispielsweise durch allmähliches Einengen der Lösung, in deren Lösungsmittel das R-Epimer schwerer löslich ist als das S-Epimer, und Abtrennen der ausfallenden Kristalle, oder durch allmähliches Zugeben eines Lösungsmittels zu einer Lösung, in dem das R-Epimer schwerer löslich ist als das S-Epimer, und Abtrennen der ausfallenden Kristalle. Als beispielhafte Lösungsmittel, in denen das R-Epimer der Verbindungen der Formel II schlechter löslich ist als das S-Epimer seien genannt: Ester, wie Ethylacetat oder Ethylacetat/Petrolether-Gemische; Alkohole, wie Ethanol oder Ethanol/Wasser-Gemische.

Durch diese fraktionierte Kristallisation, die gewünschtenfalls auch wiederholt werden kann, läßt sich erfindungsgemäß das R-Epimer zu > 97 %, insbesondere zu > 99 % anreichern.

Die saure Hydrolyse der Verbindungen der Formel II (Abspaltung des Si(R2)(R3)R4-Restes) erfolgt in an sich bekannter Weise in wäßrigen oder Wasser enthaltenden Lösungsmitteln, wie Tetrahydrofuran oder Dimethylformamid in Gegenwart einer Säure, wie Trifluoressigsäure, Essigsäure oder Chlorwasserstoff, wobei das Molverhältnis Säure/Verbindung II vorteilhafterweise zwischen 1:1 und 10:1 und das Molverhältnis Wasser/Verbindung II zwischen 5:1 und 20:1 liegt. Überraschenderweise wird bei der sauren Hydrolyse des Silylrestes der Acetalring nicht angegriffen.

Für die Durchführung des erfindungsgemäßen Verfahrens geht man vorteilhafterweise von solchen Verbindungen der Formel I aus, bei denen das R-Epimer bereits angereichert ist. Die Verbindungen der Formel I erhält man dabei in an sich bekannter Weise durch Umsetzung von 16-Hydroxyprednisolon mit dem entsprechenden Aldehyd R1-CHO, wobei durch geeignete Variation der Reaktionsbedingungen die Umsetzung so gesteuert werden kann, daß überwiegend das R-Epimer entsteht. Zur überwiegenden Herstellung des R-Epimeren der Formel I werden beispielsweise folgende Bedingungen bevorzugt: Halogenierte Kohlenwasserstoffe oder Nitromethan mit Methansulfonsäure bei RT bis 40°C, oder 35-70 %ige Perchlorsäure bei 0°C bis RT. Eine weitere Möglichkeit zur überwiegenden Herstellung des R-Epimeren besteht in der Behandlung des Epimerengemisches (Formel I) mit 70 %iger Perchlorsäure in einem geeigneten Lösungsmittel, wie z.B. Methylenchlorid, bei 0°C (Epimerisierung).

Weiterer Gegenstand der Erfindung sind die Verbindungen der Formel II, worin R1, R2, R3 und R4 die oben angegebenen Bedeutungen haben.

Die folgenden Beispiele erläutern die Erfindung näher. RT steht für Raumtemperatur, Min. für Minute(n), h für Stunde(n), Schmp. für Schmelzpunkt.

### Beispiele

### A. Herstellung der Verbindungen II

1. Verbindung IIa (R¹ = Cyclohexyl, R²=R³=R⁴=Methyl)
   3,0 g (6,37 mmol) der Verbindung I mit R¹ = Cyclohexyl werden in 15 ml Dimethylformamid gelöst, mit 510 mg (7,5 mmol) Imidazol und 980 mg (9,0 mmol) Trimethylchlorsilan versetzt und 30 Min. bei RT gerührt. Man gießt auf Natriumhydrogencarbonatlösung, saugt den Feststoff ab und spült mit Wasser nach. Rohausbeute quantitativ, R_{f} = 0,66 (Kieselgel, Ethylacetat/Petrolether = 2:3).
2. Verbindung IIb (R¹ = Cyclohexyl, R³=R⁴=Methyl, R²=Thexyl)
   10,0 g (21,2 mmol) der Verbindung 1 mit R¹ = Cyclohexyl werden in 60 ml Dimethylformamid gelöst, mit 2,0 g (29,4 mmol) Imidazol und 5,0 ml (25,4 mmol) Thexyldimethylsilylchlorid versetzt. Nach 2 h Rühren bei 30-40°C gießt man auf 400 ml 0,5N Salzsäure, saugt den Niederschlag ab und wäscht mit Wasser nach. Rohausbeute: quantitativ, R_{f} (22R) = 0,6, R_{f} (22S) = 0,56 (Kieselgel, Petrolether/Ethylacetat = 2:1).
3. Verbindung IIb (R¹ = Cyclohexyl, R³=R⁴=Methyl, R²=Thexyl)
   0,77 g (1,64 mmol) der Verbindung I mit R¹ = Cyclohexyl werden in 10,0 ml Pyridin gelöst und mit 0,45 g (2,5 mmol) Thexyldimethylchlorsilan und 10,0 mg Dimethylaminopyridin versetzt. Man erwärmt 6 h auf 80°C, gießt dann auf Wasser und extrahiert die Wasserphase mit Ethylacetat. Die organische Phase wird mit 1N Salzsäure gewaschen, mit Natriumsulfat getrocknet, abgesaugt, mit Hexan versetzt und im Vakuum langsam eingeengt. Der Niederschlag wird abgesaugt und getrocknet. Ausbeute 0,22 g (22 %); R_{f}-Wert siehe Beispiel 2.
4. Verbindung IIb (R¹ = Cyclohexyl, R³=R⁴=Methyl, R²=Thexyl)
   5 g (10,6 mmol) der Verbindung I mit R¹ = Cyclohexyl werden in 30 ml Dimethylformamid gelöst und mit 9,6 g (53,7 mmol) Thexyldimethylchlorsilan versetzt. Bei 60°C wird portionsweise 4,5 g (32,6 mmol) Kaliumcarbonat zugegeben. Nach 6 h Rühren wird mit Wasser/Ethylacetat extrahiert und die organische Phase nach Trocknen mit Natriumsulfat eingeengt. Der Rückstand wird mit 20 ml Isopropanol ausgerührt, abgesaugt und getrocknet. Ausbeute: 4,8 g (74 %); R_{f}-Wert siehe Beispiel 2.
5. Verbindung IIc (R¹=Cyclohexyl, R²=R³=R⁴=Isobutyl)
   5,0 g (10,6 mmol) der Verbindung I mit R¹ = Cyclohexyl werden in 25 ml Dimethylformamid gelöst und mit 1,0 g (14,7 mmol) Imidazol und 3,08 g (13,1 mmol) Triisobutylchlorsilan versetzt. Nach 5 h Rühren wird die Lösung in Wasser getropft, mit Ethylacetat extrahiert, die organische Phase mit Natriumsulfat getrocknet und eingeengt. Rohausbeute 93 %_{'} R_{f}=0,71 (Kieselgel, Petrolether/Ethylacetat = 3:2).
6. Verbindung IId (R¹=Cyclohexyl, R³=R⁴=Methyl, R²=t-Butyl)
   10,0 g (21,3 mmol) der Verbindung I mit R¹ = Cyclohexyl werden in 60 ml Dimethylformamid gelöst und mit 1,7 g (25,0 mmol) Imidazol und 3,77 g (25,0 mmol) t-Butyldimethylchlorsilan versetzt. Nach 3 h Rühren bei RT gießt man auf Wasser, saugt den Niederschlag ab und wäscht mit Wasser nach. Rohausbeute 95 %, R_{f}=0,76 (Kieselgel, Ethylacetat/Petrolether = 2:3).
7. Verbindung IIe (R¹=Cyclohexyl, R²=t-Butyl, R³=R⁴=Phenyl)
   4,7 g (10,0 mmol) der Verbindung I mit R¹ = Cyclohexyl werden in 25 ml Dimethylformamid gelöst und mit 885 mg (13,0 mmol) Imidazol und 3,3 g (12,0 mmol) t-Butyldiphenylchlorsilan versetzt. Nach 4 h Rühren bei RT wird die Lösung in Wasser getropft, der Niederschlag abgesaugt, mit Wasser gewaschen und getrocknet. Rohausbeute: 6,4 g (91 %): R_{f}=0,55 (Kieselgel, Petrolether/Ethylacetat = 3:2).
8. Verbindung IIf (R¹=Propyl, R²=Thexyl, R³=R⁴=Methyl)
   9,0 g (20,9 mmol) der Verbindung I mit R¹ = Propyl und 1,77 g (26,0 mmol) Imidazol werden in 50 ml Dimethylformamid gelöst und mit 4,47 ml (25,0 mmol) Thexyldimethylchlorsilan versetzt. Nach 20 Min. Rühren bei 40°C wird die Lösung in 1 l Wasser getropft, der Niederschlag abgesaugt und getrocknet. Rohausbeute: quantitativ. R_{f}=0,74 (Kieselgel, Ethylacetat/Petrolether = 1:1).

### B. Epimerenanreicherung bei den Verbindungen II

9. 1,5 g (2,76 mmol) IIa (R¹=Cyclohexyl; R²=R³=R⁴=Methyl, 92 % 22 R-Epimer) werden in der Wärme in 5 ml Ethylacetat gelöst und bis zur Trübung mit Petrolether versetzt. Die Kristalle werden abgesaugt und getrocknet. Ausbeute: 0,56 g (37 %), Schmp. 176-179°C, 96 % 22R-Epimer. R_{f}-Wert siehe Beispiel 1.
10. 11,8 g (20,2 mmol) IId (R¹=Cyclohexyl, R²=t-Butyl, R³=R⁴=Methyl, 91 % 22R-Epimer), werden in Ethylacetat gelöst und im Vakuum langsam eingeengt. Der Niederschlag wird abgesaugt und getrocknet. Ausbeute: 4,42 g (37,5 %), 98,6 % 22R-Epimer. Schmp.: 238-241°C, R_{f}-Wert siehe Beispiel 6.
11. 396 g (646 mmol) IIb (R¹=Cyclohexyl, R²=Thexyl, R³=R⁴=Methyl, 92,5,% 22R-Epimer) werden unter Erwärmen in 5,0 l Ethylacetat gelöst und im Vakuum langsam eingeengt. Die entstehende Suspension wird abgesaugt und getrocknet. Ausbeute: 317 g (80 %), 98 % 22R-Epimer. Schmp.: 237-243°C, R_{f}-Wert siehe Beispiel 2.
12. 13,0 g (21,2 mmol) IIb (R¹=Cyclohexyl, R²=Thexyl, R³=R⁴=Methyl, 91 % 22R-Epimer) werden in 200 ml abs. Ethanol umkristallisiert. Ausbeute: 8,4 g (64,6 %), 97 % 22R-Epimer. Schmp.: 232-238°C, R_{f}-Wert siehe Beispiel 2.
13. 3,0 g (4,9 mmol) IIb (R¹=Cyclohexyl, R²=Thexyl, R³=R⁴=Methyl, 93 % 22R-Epimer) werden mit 20 ml Ethylacetat heißextrahiert. Ausbeute: 2,02 g (3,29 mmol, 67,3 ), 99,3 % 22R-Epimer. Schmp.: 240-243°C, R_{f}-Wert siehe Beispiel 2.
14. 3,0 g (4,9 mmol) IIb (R¹=Cyclohexyl, R²=Thexyl, R³=R⁴=Methyl, 93 % 22R-Epimer) werden mit 20 ml Ethanol heißextrahiert. Ausbeute: 2,43 g (3,96 mmol, 81,0 ), 97,5 % 22R-Epimer. Schmp.: 241-243°C, R_{f}-Wert siehe Beispiel 2.
15. 10,0 g (17,46 mmol) IIf (R¹=Propyl, R²=Thexyl, R³=R⁴=Methyl, 82 % 22R-Epimer) werden in 22 ml Ethanol umkristallisiert. Ausbeute: 5,81g (10,1 mmol, 58,1 %), ca. 92 % 22R-Epimer. Schmp.: 220-223°C, R_{f}-Wert siehe Beispiel 7.

### C. Saure Hydrolyse der Verbindungen II

16. 16,6 g (27,1 mmol) IIb (R¹=Cyclohexyl, R²=Thexyl, R³=R⁴=Methyl, ≥ 99 % 22R-Epimer) werden in 65 ml Tetrahydrofuran gelöst, mit 4,6 g (40,5 mmol) Trifluoressigsäure und 3 ml Wasser versetzt und 12 h bei 60°C gerührt. Man versetzt mit 3,5 g festem Natriumhydrogencarbonat, saugt ab und engt das Filtrat ein. Der Rückstand wird in Ethylacetat gelöst und im Vakuum langsam eingeengt, der Niederschlag abgesaugt und getrocknet. Ausbeute 11,0 g der Verbindung I mit R¹=Cyclohexyl (86,2 %). Schmp.: 256-261°C (Heißextraktion mit Ethylacetat). 99,4 % 22R-Epimer. R_{f} = 0,21 (Kieselgel, Ethylacetat/Petrolether = 1:1).
17. 5,0 g (8,2 mmol) IIb (R¹=Cyclohexyl, R²=Thexyl, R³=R⁴=Methyl, ≥ 98 % 22R-Epimer) werden in 15 ml Dimethylformamid suspendiert, mit 1,14 g (13 mmol) Trifluoressigsäure und 2 ml Wasser und nach 6,5 h Rühren bei 50°C mit 1,1 g (13 mmol) Natriumhydrogencarbonat versetzt. Die Lösung wird in Wasser getropft, der Niederschlag abgesaugt, mit Wasser gewaschen und getrocknet. Ausbeute 3,75 g (97 %) der Verbindung I mit R¹=Cyclohexyl; ≥ 98 % 22R-Epimer, R_{f}-Wert siehe Beispiel 16.
18. 20 mg IIb (R¹=Cyclohexyl, R²=Thexyl, R³=R⁴=Methyl, ≥ 98 % 22R-Epimer) werden in 500 µl Tetrahydrofuran gelöst, mit 500 µl Essigsäure und 200 µl Wasser versetzt, 4 h bei 40°C und 12 h bei RT gerührt. DC-Umsatz vollständig, R_{f}-Wert siehe Beispiel 16, 98,5 % 22R-Epimer.
19. 20 mg IIb (R¹=Cyclohexyl, R²=Thexyl, R³=R⁴=Methyl, ≥ 98 % 22R-Epimer) werden in 1,0 ml Tetrahydrofuran gelöst und mit 50 µl 14%iger Chlorwasserstoff/Dioxanlösung versetzt. Man läßt 3 h bei RT rühren und neutralisiert dann mit Natriumhydrogencarbonat. DC-Umsatz vollständig, R_{f}-Wert siehe Beispiel 16, 98,7 % 22R-Epimer.
20. 0,5 g (0,85 mmol) IId (R¹=Cyclohexyl, R²=t-Butyl, R³=R⁴=Methyl, ≥ 97,5 % 22R-Epimer) werden in 2 ml Tetrahydrofuran gelöst und mit 200 µl ≥ (2,6 mmol) Trifluoressigsäure und 100 µl Wasser versetzt. Nach 1 h Rühren bei 70°C wird die Lösung eingeengt, der Rückstand in Diisopropylether aufgeschlämmt, abgesaugt und getrocknet. Ausbeute: 0,32 g (80 %) der Verbindung I mit R¹=Cyclohexyl; 97 % 22R-Epimer, R_{f}-Wert siehe Beispiel 16.
21. 3,2g (5,59 mmol) IIf (R¹=Propyl, R²=Thexyl, R³=R⁴=Methyl, ≥ 97 % 22R-Epimer) werden in 20 ml Tetrahydrofuran gelöst und mit 1,0 g (9,0 mmol) Trifluoressigsäure und 600 µl Wasser versetzt. Man rührt 10 h bei 65°C und 10 h bei RT, gibt 840 mg (10 mmol) Natriumhydrogencarbonat zu und arbeitet auf wie unter Beispiel 16 angegeben. Ausbeute: 1,6 g (66,5 %) der Verbindung I mit R¹=Propyl. Schmp.: 264-267°C (Heißextraktion Ethanol), 97,8 % 22R-Epimer. R_{f} = 0,19 (Kieselgel, Ethylacetat/Petrolether = 1:1).

### D. Herstellung der Ausgangsverbindungen I

22. 9,4 g (25 mmol) 16α-Hydroxyprednisolon werden in 70 ml Nitromethan suspendiert, unter Kühlung im Eisbad mit 6,87 ml (80 mmol) 70 %iger Perchlorsäure versetzt und 2,16 g (30 mmol) Butyraldehyd zugetropft. Nach 16 h Rühren bei RT wird auf Natriumhydrogencarbonatlösung gegeben, der Niederschlag abgesaugt, mit Wasser gewaschen und bei 60°C im Vakuum getrocknet. Ausbeute: 10,0 g (92 %), Epimerenverhältnis R/S = 82/18.
23. 2,0 g (5,3 mmol) 16α-Hydroxyprednisolon werden in 10 ml Nitromethan suspendiert und 1,5 ml (17,4 mmol) 50 %ige Perchlorsäure und anschließend 0,8 ml (6,6 mmol) Cyclohexanaldehyd zugetropft. Nach 2 h Rühren bei RT wird die Reaktionsmischung mit Natriumhydrogencarbonatlösung versetzt, der Niederschlag abgesaugt, mit Wasser gewaschen und bei 50°C im Hochvakuum getrocknet. Ausbeute: 2,2 g (88 %), Epimerenverhältnis R/S = 92/8.

### Bestimmung der Epimerenverhältnisse für Verbindungen I und II

Die Bestimmung der Epimerenverhältnisse erfolgt mittels HPLC. Dabei müssen Verbindungen IIa-e in analytischen Mengen nach einer der in Beispielen 16-20 beschriebenen Methoden zu den entsprechenden Verbindungen I umgesetzt und dann deren Epimerenverhältnis bestimmt werden. Dabei führen unterschiedliche Spaltungsbedingungen zu gleichen Ergebnissen.

### Chromatographiebedingungen:

Phase: ODS-Hypersil, 5 µm, d = 4,6 mm, 1 = 12,5 cm
Fluß: 1 ml/min
Raumtemperatur
Verbindung I mit R¹ = Cyclohexyl: Eluent Wasser/Ethanol = 53/47
Verbindung I mit R¹ = Propyl: Eluent Wasser/Ethanol = 65/35
Verbindung IIf: Eluent Wasser/Ethanol = 36/64

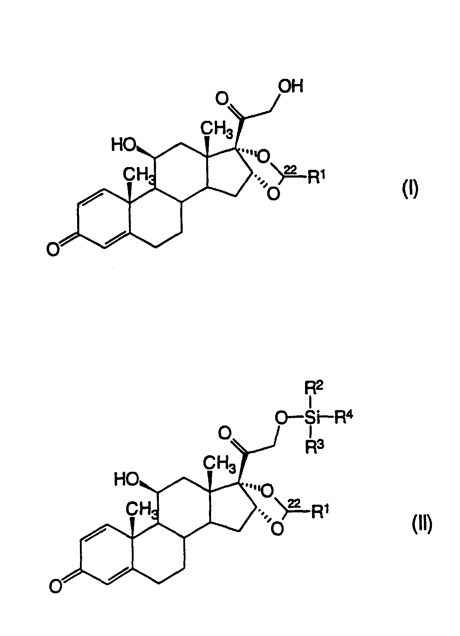

## Patentansprüche

1. Verfahren zur Anreicherung des R-Epimeren in einem R/S-Epimerengemisch von Verbindungen der Formel I, 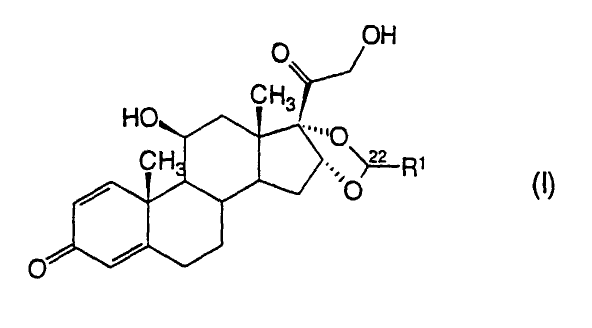 worin R1 1-7C-Alkyl oder 3-8C-Cycloalkyl bedeutet, dadurch gekennzeichnet, daß man das R/S-Epimerengemisch der Verbindungen der Formel I mit Verbindungen X-Si(R2)(R3)R4, worin R2, R3 und R4 gleich oder verschieden sind und jeweils einen 1-7C-Alkylrest oder Phenylrest darstellen und X eine geeignete Abgangsgruppe darstellt, silyliert, das erhaltene R/S-Gemisch des Silylderivats der Formel II, 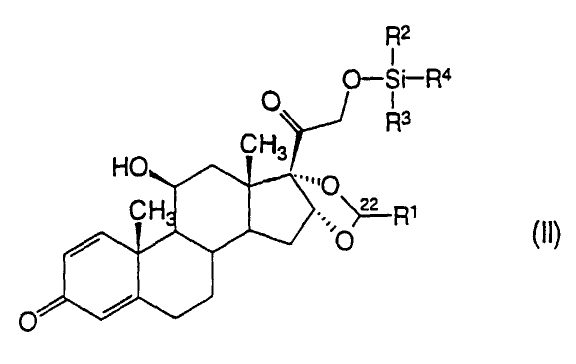 worin R1, R2, R3 und R4 die oben angegebenen Bedeutungen haben, fraktioniert zur Kristallisation bringt, und aus den zuerst erhaltenen Kristallfraktionen das mit R-Epimeren angereicherte R/S-Epimerengemisch von Verbindungen der Formel I durch saure Hydrolyse freisetzt.

2. Verfahren nach Anspruch 1, worin
R1 Propyl oder Cyclohexyl bedeutet,
R2 Methyl, Isobutyl, t-Butyl oder Thexyl bedeutet,
R3 Methyl, Isobutyl oder Phenyl bedeutet,
R4 Methyl, Isobutyl oder Phenyl bedeutet und
X Halogen darstellt.

3. Verfahren nach Anspruch 1, worin
R¹ Cyclohexyl bedeutet,
R2 Thexyl bedeutet,
R3 Methyl bedeutet,
R4 Methyl bedeutet und
X Chlor darstellt.

4. Verfahren nach Anspruch 1, wobei die fraktionierte Kristallisation in einem Lösungsmittel oder Lösungsmittelgemisch wie Ethylacetat, Ethylacetat/Petrolether-Gemisch, Ethanol oder Ethanol/Wasser-Gemisch vorgenommen wird.

5. Verfahren nach Anspruch 1, wobei die saure Hydrolyse in wäßrigen oder Wasser enthaltenden Lösungsmitteln in Gegenwart einer Säure wie Trifluoressigsäure, Essigsäure oder Chlorwasserstoff vorgenommen wird.

6. Verbindungen der Formel II 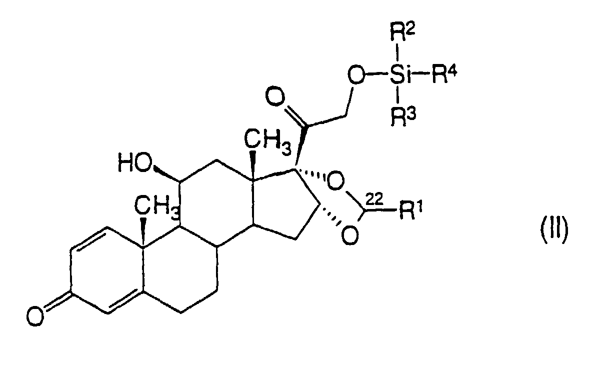 worin
R1 1-7C-Alkyl oder 3-8C-Cycloalkyl bedeutet und
R2, R3 und R4 gleich oder verschieden sind und jeweils 1-7C-Alkyl oder Phenyl bedeuten.

7. Verbindungen der Formel II nach Anspruch 6 in Form der 22R-Epimeren.

8. Verbindungen der Formel II nach Anspruch 6, worin
R1 Propyl oder Cyclohexyl bedeutet,
R2 Methyl, Isobutyl, t-Butyl oder Thexyl bedeutet,
R3 Methyl, Isobutyl oder Phenyl bedeutet und
R4 Methyl, Isobutyl oder Phenyl bedeutet.

9. Verbindung der Formel II nach Anspruch 6, worin
R1 Cyclohexyl bedeutet,
R2 Thexyl bedeutet,
R3 Methyl bedeutet und
R4 Methyl bedeutet.

10. Verbindung der Formel II nach Anspruch 6, worin
R1 Cyclohexyl bedeutet,
R2 Thexyl bedeutet,
R3 Methyl bedeutet und
R4 Methyl bedeutet
in Form des 22R-Epimeren.

## Claims

1. Process for enriching the R epimer in an R/S epimer mixture of compounds of the formula I, 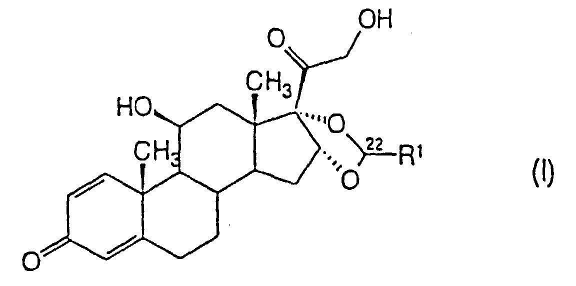 in which R1 denotes 1-7C-alkyl or 3-8C-cycloalkyl, characterized in that the R/S epimer mixture of the compounds of the formula I is silylated with compounds X-Si(R2)(R3)R4, in which R2, R3 and R4 are identical or different and each signifies a 1-7C-alkyl radical or phenyl radical and X signifies a suitable leaving group, the resulting R/S mixture of the silyl derivative of the formula II 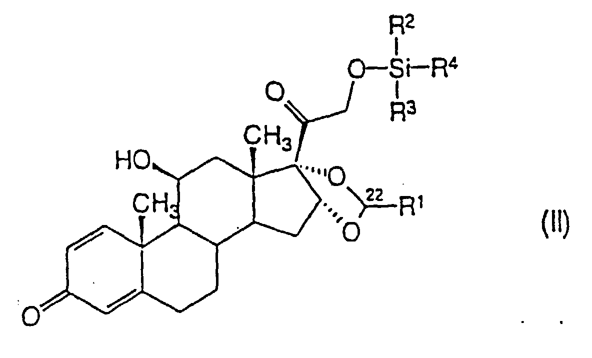 in which R1, R2, R3 and R4 have the meanings given above, is fractionally crystallized, and the R-epimer-enriched R/S-epimer mixture of compounds of the formula I is released by acid hydrolysis from the crystal fractions obtained first.

2. Process according to Claim 1, in which
R1 denotes propyl or cyclohexyl,
R2 denotes methyl, isobutyl, t-butyl or thexyl,
R3 denotes methyl, isobutyl or phenyl,
R4 denotes methyl, isobutyl or phenyl and
X signifies halogen.

3. Process according to Claim 1, in which
R1 denotes cyclohexyl,
R2 denotes thexyl,
R3 denotes methyl,
R4 denotes methyl and
X signifies chlorine.

4. Process according to Claim 1, the fractional crystallization being performed in a solvent or solvent mixture such as ethyl acetate, ethyl acetate/petroleum ether mixture, ethanol or ethanol/water mixture.

5. Process according to Claim 1, the acid hydrolysis being performed in aqueous or water-containing solvents in the presence of an acid such as trifluoroacetic acid, acetic acid or hydrogen chloride.

6. Compounds of the formula II 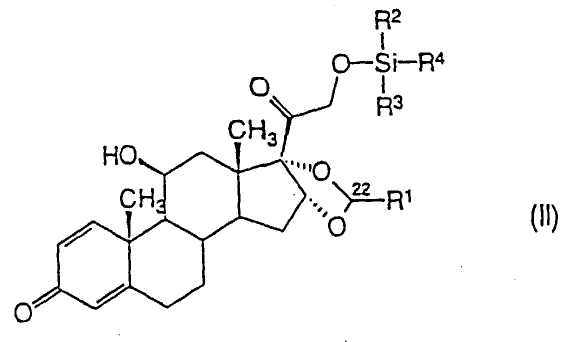 in which
R1 denotes 1-7C-alkyl or 3-8C-cycloalkyl and
R2, R3 and R4 are identical or different and each denote 1-7C-alkyl or phenyl.

7. Compounds of the formula II according to Claim 6 in the form of the 22R epimers.

8. Compounds of the formula II according to Claim 6, in which
R1 denotes propyl or cyclohexyl,
R2 denotes methyl, isobutyl, t-butyl or thexyl,
R3 denotes methyl, isobutyl or phenyl and
R4 denotes methyl, isobutyl or phenyl.

9. Compound of the formula II according to Claim 6, in which
R1 denotes cyclohexyl,
R2 denotes thexyl,
R3 denotes methyl and
R4 denotes methyl.

10. Compound of the formula II according to Claim 6, in which
R1 denotes cyclohexyl,
R2 denotes thexyl,
R3 denotes methyl and
R4 denotes methyl
in the form of the 22R epimer.

## Revendications

1. Procédé pour enrichir en épimère R un mélange d'épimères R/S de composés de formule (I) 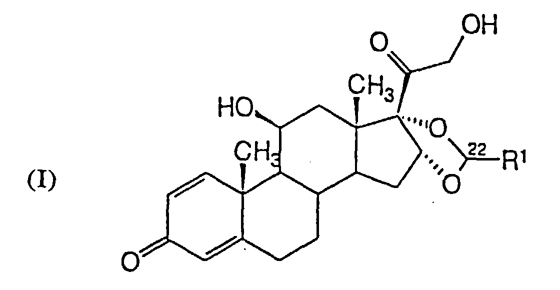 dans laquelle R¹ représente un groupe alkyle en C₁₋₇ ou cycloalkyle en C₃₋₈, caractérisé par le fait que l'on soumet le mélange d'épimères R/S des composés de formule (I) à une réaction de silylation avec des composés de formule X-Si(R²)(R³)R⁴, où R², R³ et R⁴, identiques ou différents, représentent chacun un résidu alkyle en C₁₋₇ ou phényle, et X représente un groupe partant approprié, que l'on soumet le mélange d'épimères R/S du dérivé silylé obtenu de formule (II) 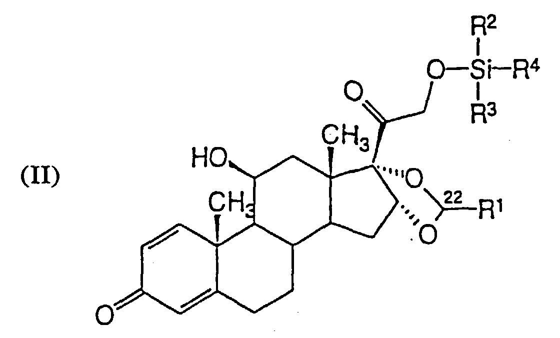 où R¹, R², R³ et R⁴ ont les significations indiquées ci-dessus, à une cristallisation fractionnée, et que l'on libère par hydrolyse acide le mélange d'épimères R/S de formule (I) enrichi en épimère R à partir des fractions ayant cristallisé en premier.

2. Procédé selon la revendication 1, dans lequel R¹ représente un groupe propyle ou cyclohexyle, R² représente un groupe méthyle, isobutyle, t-butyle ou thexyle (1,1,2-triméthyl-1-propyle), R³ représente un groupe méthyle, isobutyle ou phényle, R⁴ représente un groupe méthyle, isobutyle ou phényle et X représente un atome d'halogène.

3. Procédé selon la revendication 1, dans lequel R¹ représente un groupe cyclohexyle, R² représente un groupe thexyle, R³ représente un groupe méthyle, R⁴ représente un groupe méthyle et X représente un atome de chlore.

4. Procédé selon la revendication 1, dans lequel la cristallisation fractionnées est réalisée dans un solvant ou mélange de solvants tels que acétate d'éthyle, acétate d'éthyle/éther de pétrole, éthanol ou éthanol/eau.

5. Procédé selon la revendication 1, dans lequel l'hydrolyse acide est réalisée dans des solvants aqueux ou contenant de l'eau en présence d'un acide tel que l'acide trifluoroacétique, l'acide acétique ou l'acide chlorhydrique.

6. Composés de formule (II) 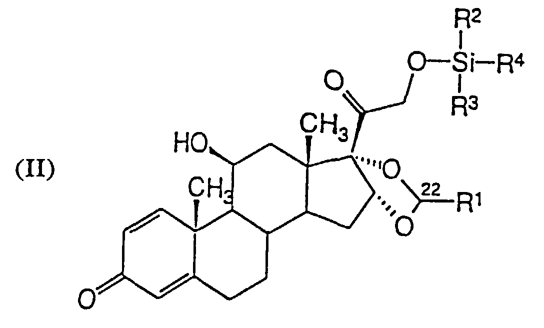 dans laquelle R¹représente un groupe alkyle en C₁₋₇ ou cycloalkyle en C₃₋₈ et R², R³ et R⁴, identiques ou différents, représentent chacun un groupe alkyle en C₁₋₇ ou phényle.

7. Composés de formule (II) selon la revendication 6 sous forme d'épimère 22R.

8. Composés de formule (II) selon la revendication 6, dans lesquels R¹ représente un groupe propyle ou cyclohexyle, R² représente un groupe méthyle, isobutyle, t-butyle ou thexyle, R³ représente un groupe méthyle, isobutyle ou phényle et R⁴ représente un groupe méthyle, isobutyle ou phényle.

9. Composés de formule (II) selon la revendication 6, dans lesquels R¹ représente un groupe cyclohexyle, R² représente un groupe thexyle, R³ représente un groupe méthyle et R⁴ représenté un groupe méthyle.

10. Composés de formule (II) selon la revendication 6, dans lesquels R¹ représente un groupe cyclohexyle, R² représente un groupe thexyle, R³ représente un groupe méthyle et R⁴ représente un groupe méthyle, sous forme d'épimère 22R.
